# EUROPEAN PATENT SPECIFICATION

(11) **EP 0 415 665 B1**
(45) Date of publication and mention of the grant of the patent: **25.01.1995**
(21) Application number: 90309306.0
(22) Date of filing: 24.08.1990
(51) Int. Cl.: A61M 39/02, A61M 25/00

(54) **Adapter for catheters**
Verbindungsstück für Katheter
Adaptateur pour cathéter

(30) Priority: 30.08.1989 US 400859
(43) Date of publication of application: 06.03.1991
(73) Proprietor: THE KENDALL COMPANY, Mansfield, Massachusetts 02048 (US)
(72) Inventor: Gross, James R., Geneva, Illinois 60134 (US)
(74) Representative: Kearney, Kevin David Nicholas

(56) References cited:
- US-A- 4 187 848
- US-A- 4 842 592

## Description

The present invention relates to adapters for connecting a catheter to a source of fluids which it is wished to introduce via the catheter or to a vessel in which it is wished to collect fluids from the catheter.

Continuous spinal anesthesia procedures and continuous epidural anesthesia procedures are of course well known in the art. In either case, the distal end of the catheter is first introduced into the patient's body with the proximal or trailing end outside the body to receive the anesthetic.

To do so, an adapter is employed connecting the proximal end of the catheter at one end of the adapter to a source of liquid anesthetic, e.g. a syringe, at the other. The adapter has a channel communicating with the catheter end so that when the liquid anesthetic is introduced into the channel, it passes through the catheter into either the subarachnoid space, if the spinal anesthesia procedure is used or into the epidural space, as would be the case with the epidural anesthesia procedure.

As an illustration of prior adapters for this purpose, mention may be made of those described and claimed in US-A-4,187,848 issued to Glenn N. Taylor. As disclosed therein, the adapter comprises two separate members, one being designated as the body member, the other being termed a compression member. The body member has an elongated bore and an opening at its distal end for receiving the proximal end of the catheter extending from the patient's body. An elongated elastic plug having a channel extending therethrough is seated in uncompressed condition within the bore of the body member, the channel being aligned with the opening of the body member so that the catheter end inserted in the opening can be positioned within the plug channel. The compression member has a port at its proximal end where the tip of a syringe may be releasably engaged for injecting liquid anesthetic. A passageway for fluid extends between the two ends of the compression member so that when the proximal end of the body member and the distal end of the compression member are secured together, the liquid anesthetic injected from the syringe may be pumped into the catheter. To connect the two members, the proximal end of the body member is provided with external threads and the distal end of the compression member with internal threads mating with the body member threads. When the threads are tightened to secure the two members, the plug is compressed to retain the catheter end positioned therein.

While a catheter adapter of this general description is in theory entirely satisfactory for connecting the catheter to the syringe, the prior art two-piece adapters currently in use, as exemplified by the teachings of the aforementioned patent, do nevertheless suffer from certain inherent disadvantages.

A primary disadvantage is that there is no locking engagement to maintain the two components together, as intended. Consequently, there is a danger of unscrewing or back-off, resulting in loss of compressive force on the plug and, consequently, in separation of the catheter from the adapter.

Another problem which occurs from time-to-time is human error in attempting to screw the two components together. It sometimes happens that one of the components is dropped. This human error necessitates the time and expense of opening a whole new sterile tray to replace the dropped component.

It is accordingly the task of this invention to provide an improved adapter which will obviate the aforementioned problems.

In accordance with the present invention, this task is solved in a simple and elegant manner by providing a unitary adapter having a first component for releasably engaging the syringe or other source of liquid medication, and a second component for securing the catheter end, the two components being attached together with the distal end of the first component in superposition with the proximal end of the second component so as to prevent accidental dropping of either component, the respective components further being movable between an open position for inserting the catheter in the adapter and a closed position for retaining the catheter in place, and means for locking the adapter in the closed position to prevent drop-off.

Thus according to the present invention an adaptor for connecting a catheter to second duct means comprises a first housing having a duct passing therethrough and affording gripping means for the catheter and a second housing having a duct passing therethrough to which the second duct means are connected, retaining means being provided to hold the first and second housing together whilst preventing their movement relative to each other from a first or open position in which the catheter may be inserted and removed to a second or closed position in which the gripping means engaged the catheter to secure it against removal, in which second position the duct in the second housing communicates with the catheter, the said retaining means consisting of snap rings (106,107) located at juxtaposed ends of the said connectors so as to engage each other when the connectors are assembled to form the unitary adapter, and in that means are provided for moving the said housings between open and closed positions, the said means being threads (108) on one end (110) of the said first housing adapted to mate with threads (112) on an end (114) of the said second housing, whereby the said housings can be screwed together to provide the said second or closed position and unscrewed to the said first or open position, and in that locking means (116,118,120) are provided for securing the said connectors in the said closed position, the said locking means comprising ratchet-type teeth (118) at the distal end of the liquid source connector (104) and a locking wing (116) on the external surface of the catheter connector housing (102), the proximal edge of the said locking wing being adapted to engage one of the said teeth when the said connectors are screwed together to the said closed or second position, the said locking wing thereby interfering with a course of the said ratchet-type teeth, and thereby preventing accidental unscrewing of the said connectors towards the said open or first position.

The invention also extends to a unitary adapter for placing the proximal end of a catheter, e.g. after it has been inserted in a patient's body, in liquid communication with a source of a liquid to be administered to a patient by means of the said catheter; the said adapter comprising: a catheter connector housing having distal and proximal ends, the said distal end having an opening through which the said proximal end of the said catheter may be inserted, the said catheter connector housing having an internal bore with which the said opening communicates; an elongated elastomeric and compressible plug adapted to be seated within the said bore adjacent the said distal end of the said catheter connector in a relatively uncompressed condition, the said plug having a channel extending therethrough adapted to be aligned with the said opening in the said distal end of the said catheter housing, whereby the said catheter inserted within the said opening can extend within the said channel, the inner dimensions of the said channel when uncompressed and of the said opening being slightly greater than the outer dimensions of the said catheter so as to permit insertion of the said catheter therewithin; a connector housing for the said liquid source means having proximal and distal ends and a passageway for the said liquid extending longitudinally between the said ends thereof, the said proximal end having means for releasably engaging the said liquid source means, the said distal end of the said source connector housing having an opening communicating with the said passageway; retaining means for retaining the said housing together with the said proximal end of the said catheter connector housing in juxtaposition to the said distal end of the the said source connector housing; means for reversibly moving the said connectors longitudinally with respect to one another from a first position permitting insertion of the said catheter into and withdrawal from the said catheter connector to a second position wherein the said passageway in the said source connector housing, the said bore in the said catheter connector, the said channel in the said plug and the said catheter when contained therein are in liquid communication to define a closed channel for administrating the said liquid from the said source means through the said catheter; and compression means for compressing the said plug when the said connector housings are in the said closed position, whereby to reduce the size of the gap of the said channel and thereby cause the said plug to frictionally engage the said catheter inserted therein and to retain the said catheter in the said adapter.

The said means for moving the said connector housings between open and closed positions are preferably threads on the said proximal end of the said catheter connector housing mating with threads on the said distal end of the said liquid source means connector housing, whereby the said housings can be screwed together to provide the said second or closed position and unscrewed to the said first or open position.

An adapter in accordance with the present invention preferably includes means for preventing overtightening of the said connectors in the said closed position.

The means for preventing overtightening may be detent means for limiting how tightly together the said housings may be screwed.

The adapter includes locking means for securing the said connectors in the said closed position, so as to prevent accidental movement toward the said open position.

The retaining means may consist of snap rings located at juxtaposed ends of the said connectors so as to engage each other when the connectors are assembled to form the unitary adapter.

One connector may have an axial tubular member which fits within a tubular portion of the other connector and the retaining means may be located between the juxtaposed surfaces of the said tubular members.

The invention may be put into practice in various ways and one specific embodiment will be described to illustrate the invention with reference to the accompanying drawings in which
Figure 1 is a fragmentary, exploded elevational view of a two-piece catheter adapter illustrative of the current state of the prior art;
Figure 2 is a perspective view illustating an adapter in accordance with the present invention;
Figure 3 is an exploded elevational view of the catheter of Figure 2; and
Figure 4 is a fragmentary top view illustrating the lock mechanism for securing the adapter in the closed position, as shown in Figure 2.

As mentioned, adapters for continuous spinal or epidural catheters currently in use are of a two-piece construction.

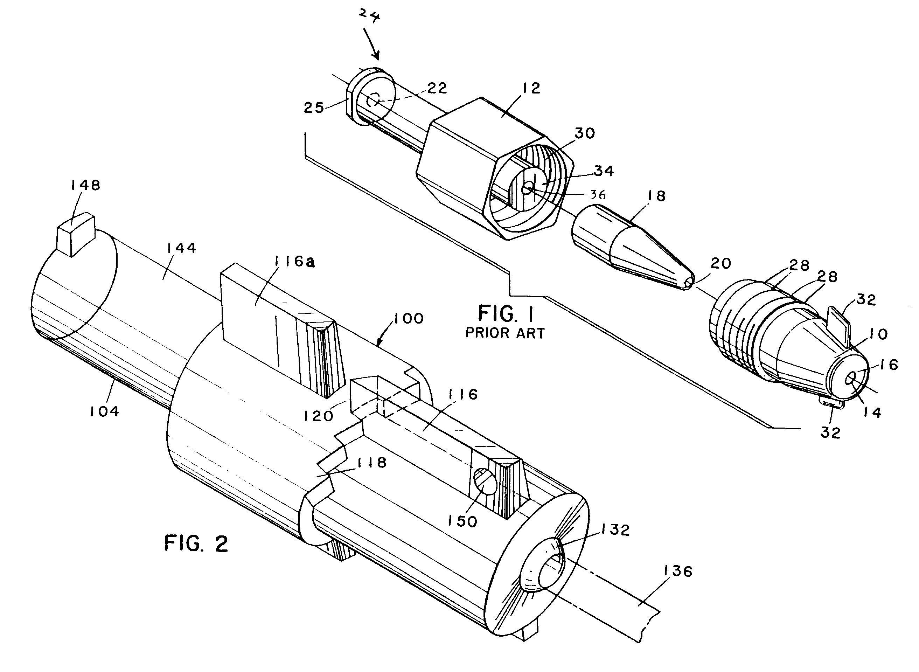

Figure 1, which need not be described in great detail for purposes of understanding the nature and objects of this invention, is illustrative of the adapters presently in use for the administration of spinal anesthesia.

As shown therein, the adapter consists of two separate body members which, for ease of reference, will be designated as catheter connector 10 and syringe connector 12. Both connectors have a longitudinally extending passageway for pumping the anesthetic from the syringe into the catheter. The catheter connector 10 has an opening 14 at its distal end 16. A compressible plug 18 of elastomeric material having a longitudinally extending channel 20 is seated within a correspondingly shaped bore within the connector 10 in a relatively uncompressed condition with channel 20 aligned with the opening 14 to receive the proximal end of a catheter (not shown) extending from the body of a patient.

The syringe connector 12 has a tapered port 22 at its proximal end 24 to receive the tip of a syringe (not shown). The proximal end 24 has luer lock flanges 25 and a female luer slip 26 (not shown) adapted to receive the luer tip of the syringe so that the syringe may be releasably locked to the connector 12.

The connector 10 has external threads 28 adjacent its proximal end which mate with internal threads 30 in the syringe connector 12 and adjacent its distal end.

When the respective connectors are screwed together, e.g. by rotating the wings 32 on the catheter connector 10, a compression collar 34 having an opening 36 compresses the plug 18 to decrease the external dimensions (gap) of the channel 20 to secure the catheter end in the adapter.

In use, the distal end of the catheter is first positioned in the patient in per se known manner. The proximal end of the catheter is inserted in the catheter connector and the two connectors are then screwed tightly together to "lock" the catheter in the adapter. The anesthesia procedure may then commence by engaging the syringe in the syringe connector and injecting the liquid anesthetic.

A major disadvantage in adapters of this general description is the tendency for back-off wherein the threads loosen or unscrew to release the compressive force sufficiently for the catheter to be displaced from the adapter. (Since the compression applied should never be so great as to materially narrow the gap of the catheter, a substantial drop in compressive force is not required for loss of the catheter connection to occur).

Another problem is the human factor involved in screwing the two separate connectors together. The parts are relatively small and extremely light. It is very easy to drop one or the other during the manual manipulative step of joining them together. If this should occur, the rigid operating room requirements for aseptic instruments demands that the dropped connector be discarded. This in turn requires the time and expense of opening a new sterile spinal tray to obtain a replacement.

The present invention is directed to a novel adapter which obviates these problems.

Stated simply, in accordance with this invention, the adapter is of a one-piece or unitary construction wherein the catheter connector and syringe connector are movably pre-connected with respect to each other from an open position to insert the catheter end and a closed position to secure the catheter in place. Anti-back-off means are provided to retain the connectors in the closed position, thereby locking the catheter end in the adapter until released by disengaging the anti-back-off means.

In the preferred embodiment, means are also provided for preventing overtightening of the connectors, which overtightening will cause excessive compressive force to be exerted on the catheter, causing unwanted lowering of its gap through which the anesthetic must traverse as well as possible damage to the catheter.

Figures 2, 3 and 4 illustrate the novel adapter of the present invention.

As shown, the adapter 100 has a catheter connector housing 102 (similar to that shown in Figure 1) and a syringe connector housing 104 which are secured in juxtaposition as a unitary device by means of an external snap ring 106 on the member 138 which engages an internal snap ring (107) (not shown) at the proximal end 110 of the catheter connector 102 to prevent separation of the respective housings.

The catheter connector 102 has a series of external threads 108 adjacent its proximal end 110 which mate with internal threads 112 at the distal end 114 of the syringe connector housing 104. By tightening or loosening the connection, e.g. by gripping one of the wings 116 or 116a and rotating the other, the connectors are movable longitudinally with respect to each other from a closed position wherein all the threads are engaged (to secure the catheter) to an open position (for insertion or removal of the catheter, as the case may be). This is achievable due to the fact that when forced the wing 116 can ride back over the teeth 118 though the teeth 118 are sufficient to prevent accidental loosening. When in the open or unthreaded position, the connectors are retained together by the snap rings 106 and 107, as previously mentioned.

As shown, a portion of the distal end 114 of the syringe connector has teeth 118. When the two connectors are screwed together, a locking wing edge 120 interferes with a course of the ratchet-type teeth 118, preventing the connectors from accidentally unscrewing and thereby releasing the catheter from the connector 102. In other words, the teeth cooperate with the locking wing edge 120 to prevent back-off and thus lock the respective connectors in the closed position until intentionally disengaged to release the catheter. To prevent overtightening which could cause detrimental excessive compression to be exerted on the catheter, a full stop detent member 122 is preferably provided.

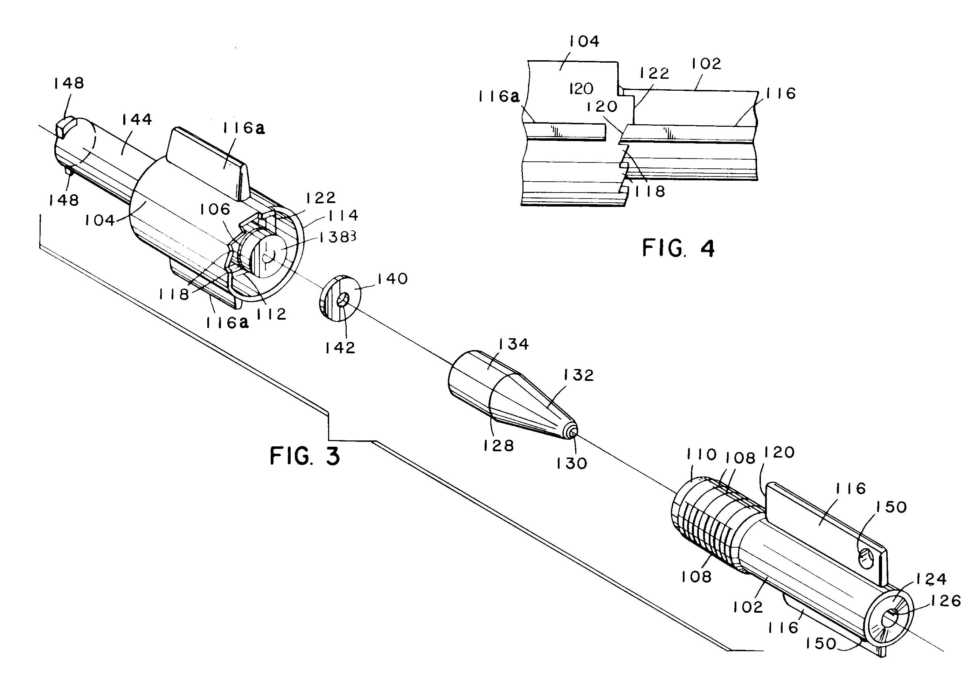

Figures 2 and 3 are of the same structure as Figure 4 but the perspective view may give the optical illusion that the teeth are not the same in the various views. For the avoidance of doubt it is confirmed that they are the same.

It will be appreciated that the teeth 118 should desirably have a rake suitable to facilitate this riding back, e.g. a rake of the order of 5°.

As was the case with the prior art adapters such as illustated in Figure 1, the catheter connector 102 is generally cylindrical and has an elongated bore. The distal end 124 has a central opening or port 126 through which the proximal (trailing) end of a catheter extending from the patient's body may be inserted.

An elongated plug 128 of elastomeric material (e.g. natural rubber, synthetic rubber, or an elastomeric polymer such as one of the KRATON (trademark of Shell Chemical Company) series having a channel or bore 130 is seated within the connector 102. The plug 128 has a distal conical section 132 and a proximal cylindrical section 134. The portion of the bore in the connector 102 in which the plug is to be received with the end of the cone adjacent the opening 126 is of like configuration so that the plug is positioned therein in relatively uncompressed condition with the channel 130 aligned with the opening 126. Both the opening 126 and the channel 130 (when the latter is uncompressed) have inner dimensions slightly larger than the outer dimensions of the catheter 136 (see Figure 2) so as to permit easy insertion through the opening and then into the plug.

When the proximal end of the catheter is so positioned, the wings 116 or 116a are rotated to place the adapter in the closed position with the locking wing edge 120 of the catheter connector 102 abutting detent member 122 of the connector 104. In this closed position, the teeth 118 lock the two connectors together to prevent loosening or back-off. In this position, the compression collar 138 located at the distal end of the syringe connector housing 104 applies compressive force to the proximal end of the plug 128, causing it to compress longitudinally to narrow the gap of the channel 130, thereby tightly frictionally engaging the catheter 130 positioned therein. To prevent damage to the proximal end of the channel 130 in the plug, a slip washer 140 is preferably provided. As seen in Figure 2, when the plug is compressed, the tip of the conical section 132 extrudes through the opening 126, thereby providing a liquid-tight strain relief collar for the catheter.

The compression collar 138 has an opening 142 communicating with a fluid passageway within the connector 104 and aligned with the washer 140 and the channel 130, thereby providing a channel for anesthetic injected into the proximal end of the syringe connector 104 to be pumped through the adapter and then into the patient via the positioned catheter for administration of the anesthesia procedure.

To accomplish the anesthesia procedure, the syringe connector 104 has a female luer slip 144 to receive the tip of the syringe and to frictionally engage the syringe luer when inserted therein. Luer locking wings 148 are provided for engagement to luer locking syringes.

The materials which may be utilized in the manufacture of the adapter of this invention will be readily apparent to those skilled in the art and as such will be a matter of individual choice. Accordingly, they are not critical and per se comprise no part of this invention. In general, with the exception of the elastomeric plug, any of the known plastics which are substantially rigid and of medical grade for use in surgical procedures may be utilized.

In like manner, the method of manufacture will be readily suggested and is accordingly not a part of this invention. Injection molding or any of the other known industrial manufacturing techniques may be employed.

It will be appreciated that the foregoing description is by way of illustration only and that various changes, modifications and additions may be made without departing from the scope of the invention herein contemplated.

For example, while not necessary in the practice of this invention, the wings 116 on connector 102 are shown to have holes 150, the purpose of which is to accommodate a safety pin, suture or other such fastening means to secure the adapter to a sheet, patient's garment or other article, if found desirable or expedient to do so.

While a toothed edge has been shown as the locking mechanism to secure the connectors against back-off, it will be appreciated that other locking means equivalent in function may be provided.

In like manner, any of the per se known means for placing the syringe or other source of fluid medication in engagement with the syringe connector may be employed, e.g. the outwardly directed luer flange described in the aforementioned US-A-4,187,848.

While reference throughout the foregoing specification has been made to anesthetic procedures utilizing a syringe to introduce liquid anesthetic into a spinal or epidural catheter, the primary task of this invention, it is to be expressly understood that the invention is not restricted to such procedures. It may be utilized in other catheterization procedures where a liquid vehicle is introduced via a catheter, which procedures require the catheter to be in fluid communication with a syringe or other source of a liquid drug or other medication.

## Claims

1. An adaptor for connecting a catheter to second duct means comprising a first housing (100) having a duct passing therethrough and affording gripping means (128) for the catheter and a second housing (104) having a duct passing therethrough to which the second duct means may be connected, means being provided for moving the said housings between open and closed positions, the said means being threads (108) on one end (110) of the said first housing adapted to mate with threads (112) on an end (114) of the said second housing, whereby the said housings can be screwed together to provide the said second or closed position and unscrewed to the said first or open position, characterised in that retaining means (106,107) are provided to hold the first and second housing together whilst permitting their movement relative to each other from a first or open position in which the catheter may be inserted and removed to a second or closed position in which the gripping means engages the catheter to secure it against removal, in which second position the duct in the second housing communicates with the catheter, the said retaining means consisting of snap rings (106,107) located at juxtaposed ends of the said connectors so as to engage each other when the connectors are assembled to form the unitary adapter, and in that and in that locking means (116,118,120) are provided for securing the said connectors in the said closed position, the said locking means comprising ratchet-type teeth (118) at the distal end of the liquid source connector (104) and a locking wing (116) on the external surface of the catheter connector housing (102), the proximal edge of the said locking wing being adapted to engage one of the said teeth when the said connectors are screwed together to the said closed or second position, the said locking wing thereby interfering with a course of the said ratchet-type teeth, and thereby preventing accidental unscrewing of the said connectors towards the said open or first position.

2. An adaptor as claimed in claim 1 in which the first housing is a catheter connector housing (100) having distal and proximal (110) ends, the said distal end having an opening through which the said proximal end of the said catheter may be inserted, the said catheter connector housing (100) having an internal bore with which the said opening communicates; the gripping means are an elongated elastomeric and compressible plug (128) adapted to be seated within the said bore adjacent the said distal end of the said catheter connector housing in a relatively uncompressed condition, the said plug having a channel (130) extending therethrough adapted to be aligned with the said opening in the said distal end of the said catheter housing, whereby the said catheter inserted within the said opening can extend within the said channel, the inner dimensions of the said channel when uncompressed and of the said opening being slightly greater than the outer dimensions of the said catheter so as to permit insertion of the said catheter therewithin; the second housing is a connector housing for the said liquid source means (104) having proximal and distal (114) ends and a passageway for the said liquid extending longitudinally between the said ends thereof, the said proximal end having means (148) for releasably engaging the said liquid source means, the said distal end of the said source connector housing having an opening communicating with the said passageway; the retaining means (106,107) retaining the said housings (100,104) together with the said proximal end of the said catheter connector housing in juxtaposition to the said distal end of the said source connector housing; the means (108,112) for moving the said housings are such that the first position permits insertion of the said catheter into and withdrawal of the said catheter from the said first housing and the second position places the said passageway in the second housing, the said bore in the said first housing, the said channel in the gripping means and the said catheter when contained therein in liquid communication to define a closed channel for administrating the said liquid from the second housing through the said catheter; and compression means for compressing the said plug when the said housings are in the said closed position, whereby to reduce the size of the gap of the said channel and thereby cause the said plug to frictionally engage the said catheter inserted therein and to retain the said catheter in the said adapter.

3. An adapter as claimed in Claim 1 or claim 2 including means (122) for preventing overtightening of the said connectors in the said closed position.

4. An adapter as claimed in Claim 3 characterised in that the means for preventing overtightening are detent means (122) for limiting how tightly together the said housings may be screwed.

5. An adapter as claimed in any one of claims 1 to 4 in which one connector has an axial tubular member which fits within a tubular portion of the other connector and the retaining means are located between the juxtaposed surfaces of the said tubular members.

## Patentansprüche

1. Adapter zur Verbindung eines Katheters mit einem zweiten Leitungsmittel, bestehend aus einem ersten Gehäuse (100), durch das hindurch ein Kanal verläuft und das ein Greifmittel (128) für den Katheter bildet, und aus einem zweiten Gehäuse (104) mit einem hindurch verlaufenden Kanal, an den das zweite Leitungsmittel anschließbar ist, aus einer Einrichtung, die zum Bewegen der Gehäuse zwischen einer Offen- und einer Schließstellung ausgebildet ist und die ein Gewinde (108) an einem Ende (110) des ersten Gehäuses ist, das mit einem Gewinde (112) an einem Ende (114) des zweiten Gehäuses zusammenpaßt, wodurch die Gehäuse zur Schaffung der zweiten bzw. Schließstellung zusammenschraubbar und in die erste bzw. Offenstellung auseinanderschraubbar sind, dadurch gekennzeichnet, daß eine Halteeinrichtung (106,107) zum Zusammenhalten des ersten und zweiten Gehäuses mit der Möglichkeit der Relativbewegung aus der ersten bzw. Offenstellung, in der der Katheter einführbar und abnehmbar ist, in die zweite bzw. Schließstellung vorgesehen ist, in der das Greifmittel zur Sicherung des Katheters gegen Abnahme am Katheter angreift, in welcher zweiten Stellung der Kanal im zweiten Gehäuse mit dem Katheter kommuniziert, daß die Halteeinrichtung aus Rastringen (106,107) besteht, die jeweils an daneben befindlichen Enden des Verbinders angeordnet sind und bei zusammengebauten Verbindern zur Bildung des einheitlichen Adapters aneinander angreifen, und daß eine Sperreinrichtung (116,118,120) zur Sicherung der Verbinder in der Schließstellung vorgesehen ist, welche Sperreinrichtung aus sperrklinkenartigen Zähnen (118) am fernliegenden Ende des Flüssigkeitsquellen-Verbinders (116) und aus einem Sperrflügel (116) an der Außenseite des Katheterverbinder-Gehäuses (102) besteht, wobei das naheliegende Rand des Sperrflügels zum Angriff an einem der Zähne ausgebildet ist, wenn die Verbinder in die Schließ- bzw. zweite Stellung zusammengeschraubt sind, wobei der Sperrflügel in die Bahn der sperrklinkenartigen Zähne eingreift und hiedurch ein zufälliges Auseinanderschrauben der Verbinder in die Offen- bzw. erste Stellung verhindert.

2. Adapter nach Anspruch 1, dadurch gekennzeichnet, daß das erste Gehäuse ein Katheterverbinder-Gehäuse (100) mit einem fernliegenden und einem naheliegenden Ende (110) ist, wobei das fernliegende Ende eine Öffnung aufweist, durch die das naheliegende Ende des Katheters einführbar ist, daß das Katheterverbinder-Gehäuse (100) eine Innenbohrung besitzt, mit der diese Öffnung kommuniziert, daß das Greifmittel ein langgestreckter, zusammendrückbarer elastomerer Stopfen (128) ist, der in relativ unzusammengedrücktem Zustand in die Bohrung neben dem fernliegenden Ende des Katheterverbinder-Gehäuses einsetzbar ist und einen durchgehenden Kanal (130) aufweist, der zum Fluchten mit der Öffnung im fernliegenden Ende des Kathetergehäuses ausgebildet ist, wodurch sich der in die Öffnung eingeführte Katheter in den Kanal erstrecken kann, wobei die Innenabmessungen des Kanals im unzusammengedrückten Zustand und diejenigen der Öffnung geringfügig größer als die Außenabmessungen des Katheters sind, um das Einführen des Katheters zu ermöglichen, daß das zweite Gehäuse ein Verbindergehäuse für das Flüssigkeitsquellenmittel (104) ist und ein naheliegendes und ein fernliegendes (114) Ende sowie einen Durchgang für die Flüssigkeit aufweist, der sich der Länge nach zwischen diesen Enden erstreckt, wobei das naheliegende Ende Mittel (148) zum lösbaren Angriff am Flüssigkeitsquellenmittel aufweist, das fernliegende Ende des Quellenverbinder-Gehäuses eine mit dem Durchgang in Verbindung stehende Öffnung aufweist, daß die Halteeinrichtung (106,107) die Gehäuse (100,104) zusammen mit dem naheliegenden Ende des Katheterverbinder-Gehäuses neben dem fernliegenden Ende des Quellenverbinder-Gehäuses festhält, daß die Einrichtung (108,112) zum Bewegen der Gehäuse derart ist, daß die erste Stellung das Einführen des Katheters in das erste Gehäuse und das Ausziehen des Katheters aus dem ersten Gehäuse ermöglicht und die zweite Stellung den Durchgang im zweiten Gehäuse, die Bohrung im ersten Gehäuse, den Kanal im Greifmittel und den Flüssigkeit enthaltenden Katheter in Strömungsverbindung anordnet, um einen geschlossenen Kanal zur Verabreichung der Flüssigkeit aus dem zweiten Gehäuse durch den Katheter zu bilden, und daß eine Druckeinrichtung zum Zusammendrücken des Stopfens vorgesehen ist, wenn sich die Gehäuse in der Schließstellung befinden, um hiedurch die Größe des Spaltes in diesem Kanal zu verringern und hiedurch den reibungsschlüssigen Angriff des Stopfens an dem eingeführten Katheter zu bewirken und den Katheter im Adapter festzuhalten.

3. Adapter nach Anspruch 1 oder 2, gekennzeichnet durch eine Einrichtung (122) zum Verhindern des Überspannens der Verbinder in der Schließstellung.

4. Adapter nach Anspruch 3, dadurch gekennzeichnet, daß die Einrichtung zum Verhindern des Überspannens Anschlagmittel (122) zur Begrenzung sind, wie fest die Gehäuse zusammengeschraubt werden können.

5. Adapter nach den Ansprüchen 1 bis 4, dadurch gekennzeichnet, daß ein Verbinder ein axiales Rohrglied aufweist, das ein einen rohrförmigen Abschnitt des anderen Verbinders paßt, und daß die Halteeinrichtung zwischen den nebeneinanderliegenden Flächen der rohrförmigen Glieder angeordnet ist.

## Revendications

1. Adaptateur pour raccorder un cathéter à des seconds moyens de conduit, comportant un premier boîtier (100) traversé par un conduit et présentant des moyens (128) d'accrochage de cathéter, et un second boîtier (104) traversé par un conduit auquel se raccordent les seconds moyens de conduit, des moyens étant prévus pour déplacer lesdits boîtiers entre la position ouverte et la position fermée, lesdits moyens étant des filets (108) sur une extrémité (110) dudit premier boîtier, adaptés pour correspondre à des filets (112) sur une extrémité (114) dudit second boitier, lesdits boîtiers pouvant être vissés ensemble pour fournir ladite seconde position ou position fermée, et dévissés jusqu'à ladite première position ou position ouverte, caractérisé en ce que des moyens de retenue (106, 107) sont prévus pour retenir ensemble le premier et le second boîtier tout en permettant leur déplacement relatif l'un par rapport à l'autre depuis une première position ou position ouverte, dans laquelle le cathéter peut être inséré et enlevé, jusqu'à une seconde position ou position fermée, dans laquelle les moyens d'accrochage accrochent le cathéter pour l'empêcher d'être enlevé, le conduit du second boîtier communiquant avec le cathéter dans cette seconde position, lesdits moyens de retenue consistant en bagues de pinçage (106, 107) situées à des extrémités juxtaposées desdits raccords, de manière à s'accrocher l'une à l'autre lorsque les raccords sont assemblés pour former l'adaptateur unitaire, et en ce que des moyens de verrouillage (116, 118, 120) sont prévus pour immobiliser lesdits raccords dans ladite position fermée, lesdits moyens de verrouillage comportant des dents (118) du type à rochet à l'extrémité distale du raccord (104) vers la source de liquide, et une aile de verrouillage (116) sur la surface extérieure du boîtier (102) de raccordement du cathéter, l'extrémité proximale de ladite aile de verrouillage étant adaptée pour engager une desdites dents lorsque lesdits raccords sont vissés ensemble vers ladite position fermée ou seconde position, ladite aile de verrouillage interférant ainsi avec un déplacement de ladite dent du type à rochet et empêchant ainsi le dévissage accidentel desdits raccords vers ladite position ouverte ou première position.

2. Adaptateur selon la revendication 1, dans lequel le premier boîtier est un boîtier de raccordement de cathéter (100) ayant une extrémité distale et une extrémité proximale (110), ladite extrémité distale ayant une ouverture à travers laquelle ladite extrémité proximale dudit cathéter peut être insérée, ledit boîtier de raccordement de cathéter (100) ayant un alésage interne avec lequel communique ladite ouverture; les moyens d'accrochage étant un bouchon allongé compressible (128) en élastomère, adapté pour être logé dans ledit alésage en position contiguë à ladite extrémité distale dudit raccordement de cathéter, dans un état relativement non comprimé, ledit bouchon étant traversé par un canal (130) adapté pour s'aligner sur ladite ouverture de ladite extrémité distale dudit boîtier de cathéter, ledit cathéter inséré dans ladite ouverture pouvant s'étendre dans ledit canal, les dimensions intérieures dudit canal non comprimé et de ladite ouverture étant légèrement plus grandes que les dimensions extérieures dudit cathéter, de manière à y permettre l'insertion dudit cathéter; le second boîtier étant un boîtier de raccordement auxdits moyens de source de liquide (104), ayant une extrémité proximale et une extrémité distale (114) et un passage pour ledit liquide s'étendant longitudinalement entre sesdites extrémités, ladite extrémité proximale ayant des moyens (148) pour accrocher de manière libérable lesdits moyens de source de liquide, ladite extrémité distale dudit boitier de raccordement de la source ayant une ouverture communiquant avec ledit passage; les moyens de retenue (106, 107) maintenant ensemble lesdits boîtiers (100, 104) et ladite extrémité proximale dudit boîtier de raccordement de cathéter en juxtaposition avec ladite extrémité distale dudit boîtier de raccordement à la source; les moyens (108, 112) pour déplacer lesdits boîtiers étant tels que la première position permet l'insertion dudit cathéter dans ledit raccord de cathéter ainsi que son extraction, et la seconde position place ledit passage dans le second boîtier, ledit alésage dans le premier boîtier, ledit canal dans les moyens d'accrochage et ledit cathéter, lorsqu'il est contenu dans ce dernier, en communication liquide, pour définir un canal fermé en vue d'administrer à travers ledit cathéter ledit liquide provenant dudit boîtier; et des moyens de compression pour comprimer ledit bouchon lorsque lesdits boîtiers de raccordement sont dans ladite position fermée, pour réduire la dimension de l'interstice dudit canal et ainsi provoquer l'engagement par friction dudit bouchon avec ledit cathéter qui y est inséré et retenir ledit cathéter dans ledit adaptateur.

3. Adaptateur selon la revendication 1 ou la revendication 2, comportant des moyens (122) pour empêcher le serrage excessif desdits raccords dans ladite position fermée.

4. Adaptateur selon la revendication 3, caractérisé en ce que les moyens pour empêcher le serrage excessif sont des moyens de butée (122) limitant la force du serrage avec laquelle lesdits boîtiers peuvent être vissés ensemble.

5. Adaptateur selon l'une quelconque des revendications 1 à 4, dans lequel un raccord présente un organe tubulaire axial qui s'adapte dans une partie tubulaire de l'autre raccord, et les moyens de retenue sont situés entre les surfaces superposées desdits organes tubulaires.
